Europäisches Patentamt

European Patent Office

Office européen des brevets

(19)

(11) Publication number: 0 395 050
A1

# EUROPEAN PATENT APPLICATION

(21) Application number: 90107926.9

(22) Date of filing: 26.04.90

(51) Int. Cl.5: C07C 51/58, C07C 55/40

(30) Priority: 27.04.89 IT 2029489

(43) Date of publication of application:
31.10.90 Bulletin 90/44

(84) Designated Contracting States:
BE DE FR GB NL

(71) Applicant: AUSIMONT S.r.l.
31, Foro Buonaparte
I-20100 Milano(IT)

(72) Inventor: Gervasutti, Claudio, Dr.
2/4, Via P. Sarpi
I-30172 Mestre, Venezia(IT)

(74) Representative: Barz, Peter, Dr.
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
D-8000 München 40(DE)

(54) Process for the preparation of perfluorosuccinyl fluoride.

(57) Perfluorosuccinyl fluoride is prepared by reaction of a mixture of 1,4-dibromo-perfluorobutane and $SO_3$ in oleum containing at least 65% of free $SO_3$ and a catalyst comprising, besides mono- and bivalent mercury sulphates, also $B_2O_3$ in quantities of from 0.3% to 8% by weight with respect to the free $SO_3$ present in the oleum during the course of the reaction.

EP 0 395 050 A1

## PROCESS FOR THE PREPARATION OF PERFLUOROSUCCINYL FLUORIDE

The present invention relates to a process for the preparation of perfluorosuccinyl fluoride (COF-$CF_2$-$CF_2$-COF). Perfluorosuccinyl fluoride is a known product used, among others, as inter-mediate in the preparation of fluoro derivatives. Methods for its preparation consist, for instance, in the electrochemical fluorination of succinyl fluoride acid, in the oxidation of perfluorocyclobutene, or in the oxidation of 1,4-diiodoper-fluorocyclobutane.

All of these processes afford very low yields of perfluorosuccinyl fluoride and are thus not suitable for being carried out on an industrial scale.

According to GB-A-2082570 perfluorosuccinyl fluoride is prepared by a process which comprises dimerization and subsequent de-halogenation of a difluoroacetyl fluoride of formula $XCF_2COF$($\underline{X}$ = Cl, Br or I) with yields of about 50%.

Said method of preparation shows, however, the drawback of being associated with very critical operational requirements, such as, e.g, anhydrous starting materials, the use of metals and high reaction temperatures which, if not strictly satisfied, result in considerably reduced yields. Moreover, the starting difluoroacetyl fluoride is difficult to prepare which further complicates said process.

It is known from US-A-3,102,139 that by oxidation of fluoroalkanes of formula RCXYZ, wherein R is an alkyl radical perhalogenated with F and/or Cl atoms; X = H, Br, Cl or F and Y and Z are Cl, Br or I; with $SO_3$ stabilized with boric anhydride, in the absence of free acids and in the presence of mercurous and mercuric sulphates fluoro-alkanoyl-monohalides may be obtained.

According to said patent, fluoroalkanes in which the reactive terminal carbon atom has attached thereto two fluorine atoms, and more particularly fluoroalkanes in which both terminal carbon atoms are monobromo-difluorinated, such as, e.g., 1,4-dibromo-perfluorobutane ($BrF_2C$-$CF_2$-$CF_2$-$CF_2Br$) cannot be converted to fluoroalkanoyl halides.

It is also known from JP-A-59/48436 to prepare perfluoroalkane dicarboxylic acid difluorides by oxidation of $\alpha,\omega$-diiodoperfluoroalkanes with $SO_3$ or oleum, in the presence of a catalyst consisting of phosphorus, molybdenum and tin, or compounds thereof.

Said method has, however, the drawback of requiring the use of iodinated perfluoroalkanes which are not easily available as industrial products and thus have to be prepared for this purpose.

Finally, it is known from US-A-4,116,977 to carry out the oxidation of $\alpha,\omega$-diiodoperfluoroalkanes with oleum at 30 - 70% of $SO_3$ in the presence of a catalyst consisting of mercury sulphate.

By means of said process only fluoroalkanes containing more than 6 carbon atoms afford high yields of fluorides of perfluorinated acids, while with those having from 3 to 5 carbon atoms, such as $\alpha,\omega$-diiodoperfluorobutane the yields are only 10 - 15%.

It has now surprisingly been found that it is possible to prepare, also by means of a continuous process, perfluorosuccinyl fluoride with high yields and conversions, by starting from 1,4-dibromo-perfluorobutane and carrying out the oxidation with sulphuric anhydride in the presence of a catalytic system comprising a mixture of mono- and di-valent mercury sulphates and boric anhydride.

More particularly, it is possible to carry out the oxidation by reacting a mixture of sulphuric anhydride and 1,4-dibromo-perfluorobutane in oleum which contains at least 65% by weight of free sulphuric anhydride as well as the components of the catalytic system in preferably dissolved form.

The reaction temperature, i.e. the temperature of the catalyst-containing oleum, preferably ranges from 45 to 70° C, particularly from 50 to 55° C.

In practice the process is carried out by introducing a mixture of 1,4-dibromo-perfluorobutane and sulphuric anhydride into oleum which contains at least 65% by weight of free sulphuric anhydride. The amount of boric anhydride in the oleum generally ranges from 0.3 to 8% by weight, preferably from 2.5 to 5.5% by weight, based on the total weight of the free sulphuric anhydride present in the oleum during the reaction.

The amounts of each the mercuric sulfate and mercurous sulphate in the oleum generally range from 0.5% to 5% b.w., based on the weight of the free sulphuric anhydride present during the reaction.

Preferably the weight ratios of $B_2O_3$/$HgSO_4$/$Hg_2SO_4$ are equal to about 10/1/1.

Thus, object of the present invention is a process for the preparation of perfluorosuccinyl fluoride, which process comprises the reaction of a mixture of 1,4-dibromo-perfluorobutane and sulphuric anhydride in oleum which contains at least 65% b.w. of free sulphuric anhydride, in the presence of a catalyst comprising mercury sulphates and 0.3 to 8% by weight, based on the weight of free sulphuric anhydride, of boric anhydride.

Preferably, the concentration of free sulphuric anhydride in the oleum during the reaction ranges from 65% to 80% by weight. The mixture of sulphuric anhydride and 1,4-dibromoperfluorobutane, which is caused to react with the oleum, preferably shows molar ratios $SO_3$/1,4-dibromoperfluoro-bu-

tane of greater than 1, particularly of from 2 to 3.

During the course of the reaction there is formed perfluorosuccinyl fluoride together with free bromine and sulphurous anhydride. The bromine may be removed by reaction with cyclohexanone and the formation of bromocyclohexane, while the sulphurous anhydride may be eliminated by reaction with potassium fluoride dissolved in distilled anhydrous diglyme.

The following example is given for purely illustrative purposes and does not in any way limit the scope of the present invention.

EXAMPLE

Into a 500 ml-reactor equipped with a stirrer and a condenser there were introduced 50 ml of oleum (65% of free sulphuric anhydride), 5 g of $B_2O_3$, 0.5 g of $Hg_2SO_4$ and 0.5 g of $HgSO_4$.

The oleum in the reactor was brought to a temperature of 50° -55° C by immersing the reactor into a Galden ® bath (perfluoropolyether) kept at a temperature of 90° C. Then 375 grams of a mixture of sulphuric anhydride and 1,4-dibromo-perfluorobutane in a weight ratio of 115:260 were added, by means of a metering cylinder and under stirring, to the oleum over about one hour.

The above mixture had been obtained by distilling sulphuric anhydride from an oleum having 65% of free sulphuric anhydride (by heating at not more than 130° C) and by then condensing and collecting the sulphuric anhydride thus obtained in a bottle containing 135 g of 1,4-dibromo-perfluorobutane.

During the reaction, under operational conditions, the temperature in the condenser was maintained at -5° C.

The reaction products flowing out of the head of the condenser were passed through an adsorption system consisting of potassium fluoride in diglyme, then through a rinsing system with cyclohexene and finally to a condensation trap maintained at -60° C by an ethanol/dry ice mixture. Under said conditions there were obtained about 120 grams of crude reaction product having 85% by weight of perfluorosuccinyl fluoride from which there were obtained, by distillation, 70 g of perfluorosuccinyl fluoride of 99% purity (yield about 50%).

**Claims**

1. Process for the preparation of perfluorosuccinyl fluoride, comprising the reaction of a mixture of 1,4-dibromoperfluorobutane with free sulphuric anhydride in oleum which contains at least 65% by weight of free sulphuric anhydride, in the presence of a catalyst comprising mercuric sulphate, mercurous sulphate and 0.3 to 8% by weight, based on the weight of the free sulphuric anhydride present in the oleum during the reaction, of boric anhydride.

2. Process according to claim 1, wherein the concentration of the free sulphuric anhydride in the oleum ranges from 65% to 80% by weight.

3. Process according to any one of claims 1 and 2, wherein the weight ratios $B_2O_3/HgSO_4/Hg_2SO_4$ equal about 10:1:1.

4. Process according to any one of claims 1 to 3, wherein the sulphurous anhydride formed during the reaction is adsorbed by a potassium fluoride solution in diglyme.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | US-A-3 102 139 (LAWLOR)<br>* column 3, line 26; column 4, lines 56,57,68-75; claim 1 * | 1 | C 07 C 51/58<br>C 07 C 55/40 |
| A | US-A-3 160 659 (DITTMAN)<br>* column 1, lines 17-21 * | 1 | |
| A | US-A-3 899 531 (SIEGEMUND)<br>* column 2, lines 36-53 * | 1 | |
| A | PATENT ABSTRACTS OF JAPAN<br>vol. 6, no. 110 (C-109)(988), 22 June 1982; & JP-A57-40434 (ASAHI GLASS) 06.03.1982 | 1 | |
| D,A | PATENT ABSTRACTS OF JAPAN<br>vol. 8. no. 138 (C-231)(1575), 27 June 1984; & JP-A-5948436 (DAIKIN KOGYO) 19.03.1984 | 1 | |
| A | FR-A-2 038 257 (KALI CHEMIE)<br>* page 6; examples 1,2 * | 1 | TECHNICAL FIELDS SEARCHED (Int. Cl.5)<br><br>C 07 C 51/00<br>C 07 C 55/00 |
| A | DE-C-1 020 970 (BAYER)<br>* column 2, lines 48-50; claims * | 1 | |

The present search report has been drawn up for all claims

| Place of search | Date of completion of the search | Examiner |
|---|---|---|
| BERLIN | 24-07-1990 | PROBERT C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                          
& : member of the same patent family, corresponding document

EPO FORM 1503 03.82 (P0401)